Europäisches Patentamt

European Patent Office

Office européen des brevets

(11) Veröffentlichungsnummer: **0 303 780**
**A2**

## (12) EUROPÄISCHE PATENTANMELDUNG

(21) Anmeldenummer: 88108654.0

(22) Anmeldetag: 31.05.88

(51) Int. Cl.⁴: **C12N 15/00 , A01H 1/00 , A01H 1/04 , A01H 1/06 , A01G 7/00 , //C12N9/00**

Patentansprüche für folgenden Vertragsstaat: ES.

Die Bezeichnung der Erfindung wurde geändert (Richtlinien für die Prüfung im EPA, A-III, 7.3).

(30) Priorität: 06.06.87 DE 3719053

(43) Veröffentlichungstag der Anmeldung:
22.02.89 Patentblatt 89/08

(84) Benannte Vertragsstaaten:
AT BE CH DE ES FR GB GR IT LI LU NL SE

(71) Anmelder: **HOECHST AKTIENGESELLSCHAFT**
**Postfach 80 03 20**
**D-6230 Frankfurt am Main 80(DE)**

(72) Erfinder: **Eckes, Peter, Dr.**
**Am Flachsland 18**
**D-6233 Kelkheim (Taunus)(DE)**
Erfinder: **Donn, Günter, Dr.**
**Sachsenring 35**
**D-6238 Hofheim am Taunus(DE)**
Erfinder: **Schulz, Arno, Dr.**
**Stauffenstrasse 22**
**D-6234 Hattersheim am Main(DE)**
Erfinder: **Wengenmayer, Friedrich, Dr.**
**Am Seyenbach 38**
**D-6238 Hofheim am Taunus(DE)**

(54) **Nutzung von pflanzenverwertbarem Stickstoff durch Kulturpflanzen mit Überexpression der Glutaminsynthetase.**

㊸57 Pflanzen, die Glutaminsynthetase überproduzieren, verwerten Stickstoff besser. Sie gedeihen daher auf Böden, die wegen eines zu geringen Angebots an pflanzenverwertbarem Stickstoff den entsprechenden "Wildtypen" kein praktisch nutzbares Wachstum ermöglichen.

EP 0 303 780 A2

FIG.1

EP 0 303 780 A2

# Verbesserte Nutzung von pflanzenverwertbarem Stickstoff durch Kulturpflanzen mit Überexpression der Glutaminsynthetase

Glutaminsynthetase ist ein pflanzliches Enzym, das eine zentrale Stellung in der Assimilation von Ammoniak und in der Regulation des Stickstoff-Metabolismus einnimmt In der Patentanmeldung WO 86/02097 (PCT) wird die Konstruktion von Pflanzenzellen bzw. Pflanzen beschrieben, die die Fähigkeit besitzen, Glutaminsynthetase in einer signifikant größeren Menge zu synthetisieren als sogenannte Wildtyp-Pflanzen. Derartige pflanzliche Zellen bzw. Pflanzen sind gegen Glutaminsynthetase-Inhibitoren, beispielsweise gegen das Herbizid Phosphinothricin, resistent.

Es wurde nun überraschend gefunden, daß selektionierte, mutagenisierte oder gentechnisch veränderte Pflanzen, die Glutaminsynthetase in größeren Mengen produzieren als Wildtyp-Pflanzen, die zum Wachsen notwendige Stickstoffquelle effizienter nutzen.

Die Erfindung betrifft somit:

ein Verfahren zur Verbesserung der Stickstoffverwertung in Kulturpflanzen, das dadurch gekennzeichnet ist, daß man in diesen Pflanzen eine Glutaminsynthetase-Überproduktion herbeiführt,

ein Verfahren zur Kultivierung von Kulturpflanzen unter Bedingungen, die arm an pflanzenverwertbarem Stickstoff sind, das dadurch gekennzeichnet ist, daß Kulturpflanzen, die Glutaminsynthetase überproduzieren, kultiviert werden,

ein Verfahren zur Erzeugung von Kulturpflanzen, das dadurch gekennzeichnet ist, daß man in diesen Pflanzen eine Glutaminsynthetase-Überproduktion herbeiführt und ihnen eine solche Menge verwertbaren Stickstoffs zuführt, die ohne Glutaminsynthetase-Überproduktion nicht zu einem nutzbaren Wachstum führte, sowie

die Verwendung der Glutaminsynthetase-Überproduktion zur Verbesserung der Nutzung von pflanzenverwertbarem Stickstoff in Kulturpflanzen.

Die Erfindung betrifft weiterhin spezielle Plasmide, die eine zur Glutaminsynthetase-Überproduktion führende Genstruktur enthalten sowie Kulturpflanzen, die aufgrund einer solchen Genstruktur Glutaminsynthetase überproduzieren.

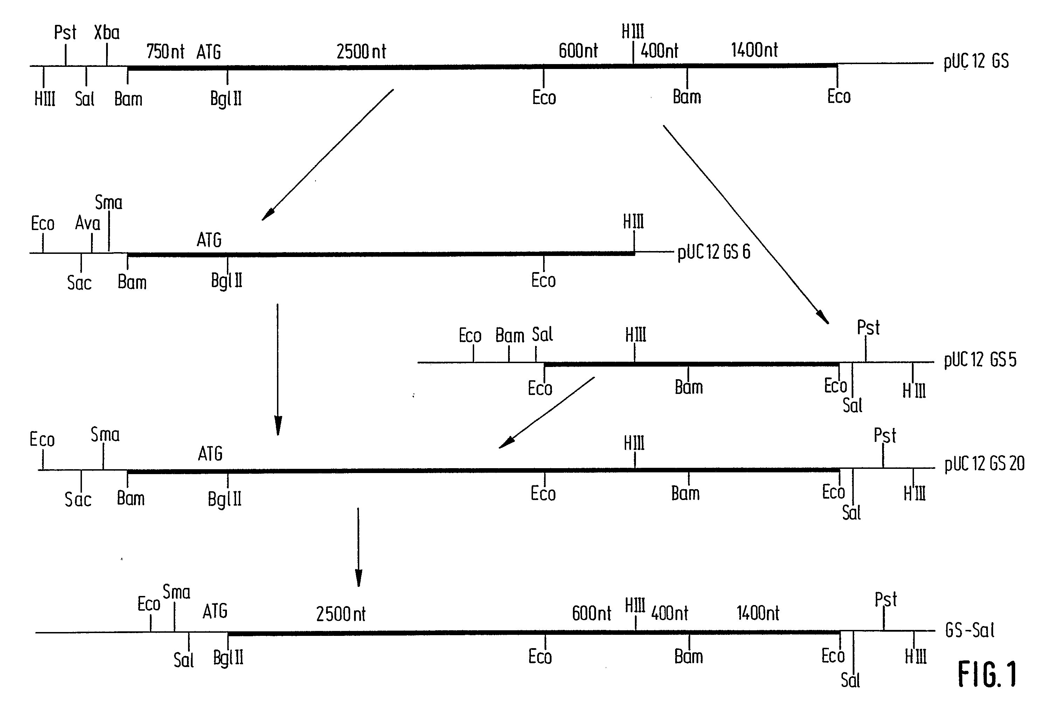

Die Figur 1 beschreibt die Überführung des natürlichen Glutaminsynthetasegens aus Medicago sativa, jeweils kloniert in den handelsüblichen Vektor pUC12, in eine um die 5′-untranslatierte Region verkürzte Form. Die Genregion ist jeweils als dicker Balken dargestellt, die Polylinker-Region des Vektors als dünne Linie. Die Restriktionsenzyme (die alle im pUC12-Polylinker Schnittstellen haben) sind teilweise abgekürzt, beispielsweise HindIII als "HIII", EcoRI als "Eco", BamHI als "Bam", weiterhin wurde bei den Enzymen Aval, Pstl, Sacl, Sall, Smal und Xbal auf die Wiedergabe des "I" verzichtet.

Im folgenden werden bevorzugte Ausführungsformen der Erfindung detailliert beschrieben.

Um die Erfindung auszuführen, kann grundsätzlich mit allen Kulturpflanzen gearbeitet werden, die Glutaminsynthetase überproduzieren. Zu dieser Gruppe von Pflanzen zählen alle diejenigen selektierten, mutierten oder gentechnisch manipulierten Kulturpflanzen, die in ihren Zellen signifikant höhere Mengen Glutaminsynthetase produzieren als der entsprechende Wildtyp. Geeignete Kulturpflanzen gehören sowohl zu den Monokotylen als auch zu den Dikotylen. Unter den Monokotylen werden bevorzugt Getreidepflanzen, insbesondere Mais, Weizen und Reis eingesetzt. Als Dikotylen werden Futterpflanzen wie Luzerne oder Klee; Tabak, Pfeffer, Erdnuß, vor allem aber Gemüsearten wie Kartoffel, Tomate; Brassica-Arten wie Kohl oder Raps; Leguminosen wie Erbsen oder Bohnen (Phaseolus, Vicia oder Vigna), Rüben wie Zuckerrübe, Rote Beete oder Karotte, sowie Sojabohne verwendet.

Wie schon erwähnt wird in der Patentanmeldung WO 86/02097 die Konstruktion von Pflanzenzellen bzw. Pflanzen beschrieben, die in der Lage sind, Glutaminsynthetase in größeren Mengen zu produzieren. Derartig hergestellte bzw. aus pflanzlichen Zellkulturen regenerierte Pflanzen können erfindungsgemäß eingesetzt werden. Geeignet sind sowohl Pflanzen, in denen durch entsprechenden Selektionsdruck das in der Pflanze bzw. pflanzlichen Zelle natürlich vorkommende Glutaminsynthetase-Gen in einer höheren Kopienzahl gebildet wird, als auch mit herkömmlichen Mitteln mutagenisierte oder gentechnisch veränderte Pflanzen, die mit Hilfe verschiedener Methoden hergestellt werden können. Beispielsweise kann eine Pflanzenzelle mit folgender Gen-Kombination konstruiert werden:

a) eine genetische Sequenz, die für Glutaminsynthetase kodiert und in der pflanzlichen Zelle exprimiert wird, kann mit

b) einer zweiten genetischen Sequenz verknüpft werden, die in der Lage ist, die Expression der unter a) genannten genetischen Sequenz zu erhöhen, d.h. die Menge an aktiver Glutaminsynthetase in der Zelle signifikant zu steigern.

Unter einer signifikanten Steigerung der Menge an aktiver Glutaminsynthetase ist diejenige Steigerung

2

zu verstehen, die für die jeweilige Pflanze ausreicht, um, im Hinblick auf die Menge an pflanzenverwertbarem Stickstoff, auch unter Bedingungen wachsen können, die für den Wildtyp nur zu vermindertem, für praktische Zwecke unbrauchbarem Wachstum führen.

Eine derartige Gen-Kombination kann direkt in das Genom der Pflanzenzelle oder mit Hilfe eines Vektors, bevorzugt ist hier das Ti-Plasmid aus Agrobacterium tumefaciens, eingeschleust werden. Dabei kann die genannte Glutaminsynthetase-Gen-Sequenz für die Pflanze bzw. die Zellkultur sowohl homolog als auch heterolog sein. Im Falle einer heterologen Gen-Sequenz kann diese sowohl von anderen Organismen, beispielsweise Mikroorganismen oder Tieren, als auch vorzugsweise von anderen Pflanzenspezies stammen. Bevorzugte Pflanzen, aus denen das Glutaminsynthetase-Gen gewonnen werden kann, sind beispielsweise Arabidopsis thaliana, Phaseolus vulgaris, Medicago sativa, Nicotiana plumbagenifolia, Pisum sativum oder Nicotiana tabacum.

Die zweite Gensequenz kann eine Promotor-Sequenz sein, und zwar die Sequenz eines in Pflanzen hoch exprimierenden Promotors. Ein derartiger Promotor muß lediglich eine wichtige Bedingung erfüllen, nämlich daß er in Kombination mit dem Glutaminsynthetase-Gen das Enzym in den pflanzlichen Zellen in signifikant größeren Mengen exprimiert als es in dem nicht veränderten Wildtyp der Fall ist. Der Promotor kann für die Wirtszelle sowohl homolog als auch heterolog sein. Im Falle von heterologen Promotoren werden vorzugsweise Gensequenzen von anderen Pflanzenspezies als auch von Mikroorganismen, die in Symbiose mit Pflanzen leben, oder von Pflanzenviren verwendet.

Geeignet sind beispielsweise der Promotor der kleinen Untereinheit (small subunit, ss) der Ribulose-Biphosphat-Carboxylase, sowie der Promotor des Chlorophyll a/b bindenden Proteins [WO 86/02097; A. Cashmore, Genetic Engineering of Plants, An Agricultural Perspective, Plenum, New York 1983, Seiten 29-38; Coruzzi G. et al., The Journal of Biological Chemistry 258, 1399 (1983); Dunsmuir, P., et al., Journal of Molecular and Applied Genetics 2, 285 (1983)].

Vorzugsweise werden jedoch die Promotoren folgender Gene eingesetzt:

a) Gen 1' der TR-DNA aus Agrobacterium tumefaciens A6 (Velten et al., EMBO J. 3, 2723 (1984))

b) Gen 2' der TR-DNA aus Agrobacterium tumefaciens A6 (Velten et al., a. a. O.)

c) ST-LS1, ein blatt- bzw. stengelspezifisches und lichtabhängig reguliertes Gen aus Solanum tuberosum (Eckes et al., Mol. Gen. Genet. 205, 15 (1986))

d) 35S-Transkript aus Cauliflower Mosaic Virus (Pietrzak et al., Nucl. Acids Res. 14, 5857 (1986)).

Die mit diesen Promotoren erhaltenen Genkonstruktionen sind neu und ebenfalls ein Bestandteil der Erfindung.

Besonders bevorzugt wird an diese Promotoren das von Tischer et al. (Mol. Gen. Genet. 203, 221 (1986)) beschriebene (bzw. in der EP-A 0 239 801 vorgeschlagene) Gen der Glutaminsynthetase aus Medicago sativa gekoppelt, nachdem es durch verschiedene gentechnische Methoden wie Restriktionsspaltungen, Ligationen etc. (Maniatis et al., Molecular Cloning, Cold Spring Harbor, 1982) entsprechend verändert wurde. Es können aber auch Glutaminsynthetase-Gene aus anderen Pflanzenspezies zusammen mit diesen Promotoren Anwendung finden. Ferner können mutierte Gene Verwendung finden, wie sie in der EP-A 0 240 792 vorgeschlagen wurden.

Derartige Promotor-Glutaminsynthetasegen-Konstruktionen können nach an sich bekannten Methoden sowohl in entsprechende pflanzliche Zellkulturen oder Protoplasten, aus denen dann jeweils Pflanzen regeneriert werden können, als auch in die fertige Kulturpflanze gebracht werden [Potrykus et al., Plant Mol. Biol. Reporter 3, 117 (1985); Horsch et al., Science 227, 1229 (1985)].

Pflanzen mit erhöhter Glutaminsynthetase-Produktion kann man ebenfalls durch Mutagenisierung der Wildtyppflanzen erhalten. Die Mutagenisierung kann sowohl mit Chemikalien geschehen, wie beispielsweise Ethylmethansulfonat oder Nitrosoguanidin, oder auch durch Bestrahlung, wie z.B. mit Röntgen- oder UV-Strahlen. Man verwendet derartige Mutagene in derartigen Dosen, so daß ca. 30 bis 70 % der Zellen abgetötet werden.

Die mutierten oder transformierten Pflanzen zeigen dann ein signifikant schnelleres Wachstum unter stickstoffarmen Wachstumsbedingungen als die herkömmlichen Wildtyp-Pflanzen. Unter stickstoffarmen Wachstumsbedingungen werden Bedingungen verstanden, unter denen der Wildtyp nur ein stark vermindertes Wachstum zeigt. Bei den Leguminosen kann sich eine Erhöhung der Glutaminsynthetase, die durch Symbiose mit Knöllchenbakterien und Selektion, Mutagenisierung oder gentechnologische Veränderung hervorgerufen werden kann, im Hinblick auf die Stickstoffverwertung besonders positiv auswirken.

Die Pflanzen können sowohl auf dem Feld als auch auf künstlichen Nährböden bei Tageslicht oder auch mit Beleuchtung von ca. 3000 bis 6000 Lux, bevorzugt ca. 4000 Lux, bei einem Rhythmus von 16 Stunden Helligkeit und 8 Stunden Dunkelheit, kultiviert werden. Zweckmäßig geschieht dies bei Temperaturen von 15 bis 40 °C, bevorzugt bei 20 bis 30 °C. Dabei spielt es keine Rolle, ob der pflanzenverwertbare Stickstoff aus der Luft aufgenommen wird oder in Form von Nitrit oder Nitrat aus dem Nährboden bezogen wird.

Die angegebenen Bedingungen stellen nur Näherungswerte dar, da erfindungsgemäß Kulturpflanzen unterschiedlicher Art eingesetzt werden können, die natürlich auch von einander verschiedene Wachstumsbedingungen aufweisen können. Diese sind dem Fachmann jedoch bekannt oder können in einfachen Vorversuchen festgelegt werden.

In den folgenden Beispielen wird die Erfindung weitergehend erläutert.

**Beispiel 1**

Die einzelnen Klonierungsschritte des Glutaminsynthetase-(GS-) Gens aus Medicago sativa [Tischer et al., a. a. O.] sind in Fig. 1 dargestellt. Die oberste Zeile der Figur stellt einen Ausschnitt aus dem Plasmid pUC 12 GS dar, bei dem das GS-Gen zwischen die BamHI- und EcoRI-Stellen von pUC12 kloniert ist. Hierbei wird von einer EcoRI-Schnittstelle in der 3´-untranslatierten Region Gebrauch gemacht, die in der Tabelle in Tischer et al., a. a. O., nicht mehr wiedergegeben ist. pUC 12 GS wird erhalten, wenn man das dort beschriebene 4,2 kb BamHI-Fragment und das 1,6 kb BamHI-EcoRI-Fragment zusammen in pUC12 kloniert.

In der zweiten Zeile der Figur ist das aus pUC 12 GS durch Schneiden mit BamHI und HindIII und Klonieren des 3,85 kb Fragments erhaltene Plasmid pUC 12 GS 6 (ausschnittsweise) dargestellt.

In der dritten Zeile der Figur ist ein Ausschnitt aus dem Plasmid pUC 12 GS 5 dargestellt. Dieses Plasmid erhält man aus pUC 12 GS durch Schneiden mit EcoRI, Anfügen eines SalI-Linkers an das isolierte 2,4 kb Fragment und Klonieren in die SalI-Schnittstelle von pUC12.

In der vierten Zeile der Figur ist ein Ausschnitt aus dem Plasmid pUC 12 GS 20 gezeichnet. Dieses Plasmid erhält man durch Öffnen von pUC 12 GS 6 mit HindIII und Ligieren mit dem 1,8 kb HindIII-Fragment aus pUC 12 GS 5.

In der letzten Zeile der Figur findet sich ein Ausschnitt aus dem Plasmid GS-Sal. Dieses erhält man durch Schneiden von pUC 12 GS 20 mit SmaI und BglII, wobei das 0,75 kb Fragment eliminiert wird, das u. a. für die ersten fünf Aminosäuren der GS kodiert. Hierfür wird der folgende SalI-BglII-Adapter

```
                         Met   Ser   Leu   Leu   Ser  (Asp)
    TC   GAC   CAA   AAC   ATG   TCT   CTC   CTT   TCA
         G     GTT   TTG   TAC   AGA   GAG   GAA   AGT   CTA   G
    (SalI)                                        (BglII)
```

eingesetzt, der die Kodons für diese fünf Aminosäuren wieder ergänzt.

Aus GS-Sal wird nun das GS-Gen, beginnend mit dem 6. Nukleotid vor dem Translationsstartcodon ATG und endend hinter einem ca. 1200 Nukleotide langen 3´ nichttranslatierten Bereich, mit dem Restriktionsenzym SalI als einzelnes Fragment isoliert.

Die Promotoren folgender Gene werden an das Glutaminsynthetase-Gen gekoppelt:

a) Gen 1´ der TR-DNA aus Agrobacterium tumefaciens A6 (Velten et al., EMBO J. 3, 2723 (1984))

b) Gen 2´ der TR-DNA aus Agrobacterium tumefaciens A6 (Velten et al., EMBO J. 3, 2723 (1984))

c) ST-LS1, ein blatt- bzw. stengelspezifisches und lichtabhängig reguliertes Gen aus Solanum tuberosum (Eckes et al., Mol. Gen. Genet. 205, 15 (1986))

d) 35S-Transkript aus Cauliflower Mosaic Virus (Pietrzak et al., Nucl. Acids Res. 14, 5857 (1986)).

Die Promotoren der unter a und b genannten Gene werden in das für die spätere Pflanzentransformation nützliche Plasmid pPCV 701 kloniert (Koncz et al., Proc. Natl. Acad. Sci., USA 84, 131 (1987)). Der ST-LS 1 Promotor wird als ca. 1600 bp langes EcoRI-MboII-fragment (über EcoRI-SmaI in den Polylinker des intermediären E. coli Vektors pMPK 110 kloniert (Peter Eckes, Dissertation Univers. Köln, 1985). Der 35 S-Promotor wird als ca. 540 bp langes EcoRI-SmaI Fragment (über EcoRI-SmaI) ebenfalls in pMPK 110 kloniert.

Hinter den unter a, c und d genannten Promotoren mit den im folgenden aufgeführten Insertionssequenzen befindet sich jeweils eine SalI-Schnittstelle, so daß das modifizierte GS-Gen in diese Schnittstelle in richtiger Orientierung inseriert werden kann.

Die Fusionsstellen zwischen den Promotoren und dem modifizierten GS-Gen ergeben sich wie folgt:

TR1:

    Promotor    AAACACCGATATTCATTAATCTTATCTAGTTTCTCAAAAAAA
400 Nukleotide


                TTCATATCTTCCACACGTGGATCGATCCGTCGAC....
        .                                    (SalI)

ST-LS1:

    Promotor    AAGAAGAAAAAAGGTGGGGATCCGTCGAC...
    ca. 1600 Nukleotide              ↑         (SalI)

                                (MboII/SmaI)

35S:

    Promotor       GGGTACCCGGGGATCCTCTAGAGTCGAC...
    ca. 500 Nukleotide   (SmaI)            (SalI)

Hinter dem unter b genannten Promotor ist eine BamHI-Restriktionsschnittstelle. Nach der Restriktionsverdauung werden die entstandenen überstehenden DNA-Enden mit dem Enzym DNA-Polymerase (Klenow-Fragment) aufgefüllt, ebenso wie die SalI-Enden des GS-Gens. Über die so entstandenen stumpfen Enden der DNA-Fragmente (blunt ends) wird das GS-Gen hinter den TR2-Promotor ligiert.

Die so entstandenen Promotor-GS-Gen-Konstrukte c und d werden mit Hilfe des intermediären E. coli-Vektors pMPK110 in Agrobacterium tumefaciens überführt (Peter Eckes, Dissertation, Univ. Köln, S. 91 f (1985)). Diese sogenannte Konjugation wird nach dem von Van Haute et al., EMBO J. 2, 441, (1983) beschriebenen Verfahren durchgeführt. Dabei wird das Gen mit seinen Regulationssignalen durch homologe Rekombination über die im pMPK110-Vektor und im Ti-Plasmid pGV3850 Km (Jones et al., EMBO J. 4, 2411 (1985)) enthaltenen Sequenzen des Vektors pBR322 in das Ti-Plasmid integriert. Auf dem Ti-Plasmid pGV3850Km ist nun neben dem schon vorher vorhandenen, in Pflanzen wirksamen Resistenzgen gegen das Antibiotikum Kanamycin auch das Promotor-GS-Gen-Konstrukt lokalisiert. Beide Gene werden mittels der sogenannten "leaf disc"-Transformationsmethode in Tabakpflanzen übertragen (Horsch et al., Science 227, 1229 (1985)).

Die Promotor-GS-Gen-Konstrukte a und b auf dem Transformationsplasmid pPCV 701 (s.o.) werden nach der von Koncz et al. (Mol. Gen. Genet 204, 383 (1986)) beschriebenen Methode in Agrobacterium tumefaciens pGV3101 mobilisiert. Mittels der oben genannten "leaf disc"-Methode werden die Promotor-GS-Gen-Konstrukte in Agrobacterium zusammen mit dem bereits auf dem Plasmid pPCV 701 lokalisierten Kanamycin-Resistenz-Gen als selektierbarem Marker in Tabakpflanzen übertragen.

Transformierte Sprosse werden aufgrund der mitübertragenen Resistenz gegen das Antibiotikum Kanamycin selektioniert und zu vollständigen Pflanzen regeneriert. Durch DNA-Analyse ("Southern Blotting"), RNA-Analyse ("Northern Blotting") und Protein-Analyse ("Western Blotting") der transformierten Pflanzen wird das Vorhandensein und die Expression des GS-Gens nachgewiesen.

Auf der RNA-Ebene wird das übertragene GS-Gen aus Medicago sativa ca. 10 mal stärker in transformierten Tabakpflanzen exprimiert, als in Medicago selbst. Das GS-Protein macht in transformierten Pflanzen bis zu 5 % des Gesamtproteins aus. Die Glutaminsynthetaseaktivität ist ebenfalls erhöht, wie aus der folgenden Tabelle hervorgeht:

Tabelle

| Spezifische Glutaminsynthetase-Aktivität in Tabak | |
|---|---|
| Typ | Aktivität (nkat/mg Gesamtprotein) |
| Tabak-Blatt (Wildtyp) | 4 |
| Tabak-Blatt (hergestellt nach Beispiel 1) | 20 |

**Beispiel 2**

Gemäß Beispiel 1 transformierte und nichttransformierte Tabakpflanzen werden in dem (handelsüblichen) Kultursubstrat Perlit kultiviert. Als Nährlösung dient das Hoagland-Medium [Hoagland et al., Proc. Am. Soc. Hort. Sci. 30, 288 (1933)], bei dem der Nitratgehalt auf 0,5 mM reduziert wurde. Glutaminsynthetase überproduzierende Pflanzen zeigen ein um ca. 20 % und damit signifikant schnelleres Wachstum als nicht transformierte Pflanzen.

Das gleiche Ergebnis wird bei Kultivierung der Pflanzen auf Murashige-Skoog-Medium (Physiologia Plantarum 15, 473 (1962)) erhalten, das nur 0,5 mM $KNO_3$ enthält.

**Ansprüche**

1. Verfahren zur Verbesserung der Stickstoffverwertung in Kulturpflanzen, dadurch gekennzeichnet, daß man in diesen Pflanzen eine Glutaminsynthetase-Überproduktion herbeiführt.

2. Verfahren zur Kultivierung von Kulturpflanzen unter Bedingungen, die arm an pflanzenverwertbarem Stickstoff sind, dadurch gekennzeichnet, daß Kulturpflanzen, die Glutaminsynthetase überproduzierenden, kultiviert werden.

3. Verfahren zur Erzeugung von Kulturpflanzen, dadurch gekennzeichnet, daß man in diesen Pflanzen eine Glutaminsynthetase-Überproduktion herbeiführt und ihnen eine solche Menge verwertbaren Stickstoff zuführt, die ohne Glutaminsynthetase-Überproduktion nicht zu einem nutzbaren Wachstum führt.

4. Verfahren nach einem oder mehreren der Ansprüche 1 bis 3, dadurch gekennzeichnet, daß die Glutaminsynthetase-Überproduktion durch Selektion, Mutagenisierung oder genetische Manipulation der Pflanzen bzw. Pflanzenzelle herbeigeführt wird.

5. Verfahren nach Anspruch 4, dadurch gekennzeichnet, daß die Glutaminsynthetase-Überproduktion durch Einschleusen einer Glutaminsynthetasegen-Promotor-Kombination in die Pflanzenzelle herbeigeführt wird.

6. Verfahren nach Anspruch 5, dadurch gekennzeichnet, daß ein aus Arabidopsis thaliana, Phaseolus vulgaris, Nicotiana plumbagenifolia, Pisum sativum, Nicotiana tabacum und vorzugsweise aus Medicago sativa gewonnenes Glutaminsynthetase-Gen verwendet wird.

7. Verfahren nach Anspruch 5 oder 6, dadurch gekennzeichnet, daß ein hoch exprimierender, aus Pflanzen oder aus Mikroorganismen, die in Symbiose mit Pflanzen leben, oder aus Pflanzenviren gewonnener Promotor verwendet wird, vorzugsweise der Promotor zu einem der folgenden Gene
    a) Gen 1´ oder Gen 2´ der TR-DNA aus Agrobacterium tumefaciens A6,
    b) ST-LS1 aus Solanum tuberosum oder
    c) 35S-Transkript aus Cauliflower Mosaic Virus.

8. Verwendung der Glutaminsynthetase-Überproduktion zur Verbesserung der Nutzung von pflanzenverwertbarem Stickstoff in Kulturpflanzen.

9. Plasmid, vorzugsweise ein Derivat des Plasmids pPCV 701 oder pMPK 110, enthaltend das Glutaminsynthetase-Gen aus Medicago sativa und den Promotor zu einem der folgenden Gene:
    a) Gen 1´ oder Gen 2´ der TR-DNA aus Agrobacterium tumefaciens A6,
    b) ST-LS1 aus Solanum tuberosum oder
    c) 35S-Transkript aus Cauliflower Mosaic Virus.

10. Kulturpflanzen mit verbesserter Nutzung des pflanzenverwertbaren Stickstoffs, enthaltend ein Strukturgen der Glutaminsynthetase, vorzugsweise aus Medicago sativa, das durch einen in Pflanzen hoch exprimierenden Promotor zu einem der folgenden Gene

6

a) Gen 1′ oder Gen 2′ der TR-DNA aus Agrobacterium tumefaciens A6,

b) ST-LS1 aus Solanum tuberosum oder

c) 35S-Transkript aus Cauliflower Mosaic Virus,

gesteuert wird.

Patentansprüche für folgenden Vertragsstaat : ES

1. Verfahren zur Verbesserung der Stickstoffverwertung in Kulturpflanzen, dadurch gekennzeichnet, daß man in diesen Pflanzen eine Glutaminsynthetase-Überproduktion herbeiführt.

2. Verfahren zur Kultivierung von Kulturpflanzen unter Bedingungen, die arm an pflanzenverwertbarem Stickstoff sind, dadurch gekennzeichnet, daß Kulturpflanzen, die Glutaminsynthetase überproduzieren, kultiviert werden.

3. Verfahren zur Erzeugung von Kulturpflanzen, dadurch gekennzeichnet, daß man in diesen Pflanzen eine Glutaminsynthetase-Überproduktion herbeiführt und ihnen eine solche Menge verwertbaren Stickstoff zuführt, die ohne Glutaminsynthetase-Überproduktion nicht zu einem nutzbaren Wachstum führt.

4. Verfahren nach einem oder mehreren der Ansprüche 1 bis 3, dadurch gekennzeichnet, daß die Glutaminsynthetase-Überproduktion durch Selektion, Mutagenisierung oder genetische Manipulation der Pflanzen bzw. Pflanzenzelle herbeigeführt wird.

5. Verfahren nach Anspruch 4, dadurch gekennzeichnet, daß die Glutaminsynthetase-Überproduktion durch Einschleusen einer Glutaminsynthetasegen-Promotor-Kombination in die Pflanzenzelle herbeigeführt wird.

6. Verfahren nach Anspruch 5, dadurch gekennzeichnet, daß ein aus Arabidopsis thaliana, Phaseolus vulgaris, Medicago sativa, Nicotiana plumbagenifolia, Pisum sativum oder Nicotiana tabacum gewonnenes Glutaminsynthetase-Gen verwendet wird.

7. Verfahren nach Anspruch 6, dadurch gekennzeichnet, daß das Glutaminsynthetase-Gen aus Medicago sativa verwendet wird.

8. Verfahren nach Anspruch 5, dadurch gekennzeichnet, daß ein hoch exprimierender, aus Pflanzen oder aus Mikroorganismen, die in Symbiose mit Pflanzen leben, oder aus Pflanzenviren gewonnener Promotor verwendet wird.

9. Verfahren nach Anspruch 8, dadurch gekennzeichnet, daß der Promotor zu einem der folgenden Gene verwendet wird:

a) Gen 1′ oder Gen 2′ der TR-DNA aus Agrobacterium tumefaciens A6,

b) ST-LS1 aus Solanum tuberosum oder

c) 35S-Transkript aus Cauliflower Mosaic Virus.

10. Verwendung der Glutaminsynthetase-Überproduktion zur Verbesserung der Nutzung von pflanzenverwertbarem Stickstoff in Kulturpflanzen.

FIG.1

EP 0 303 780 A2